# EUROPEAN PATENT SPECIFICATION

(11) **EP 2 502 928 B1**
(45) Date of publication and mention of the grant of the patent: **08.11.2017**
(21) Application number: 10831394.1
(22) Date of filing: 24.09.2010
(51) Int. Cl.: A23L 33/10, A23L 33/12, A23D 7/01, A23L 2/52, A23J 7/00, A61K 8/00, A61Q 19/00, A61K 8/98, C07F 9/10, A61K 9/127, A61K 8/55

(54) **PROCESS FOR PRODUCTION OF PURIFIED EGG-YOLK PHOSPHOLIPID COMPOSITION, AND PHARMACEUTICAL COMPOSITION, COSMETIC COMPOSITION OR FOOD COMPOSITION COMPRISING PURIFIED EGG-YOLK PHOSPHOLIPID COMPOSITION PRODUCED BY THE PROCESS**
VERFAHREN ZUR HERSTELLUNG EINER GEREINIGTEN EIGELB-PHOSPHOLIPID-ZUSAMMENSETZUNG SOWIE PHARMAZEUTISCHE ZUSAMMENSETZUNG, KOSMETISCHE ZUSAMMENSETZUNG ODER LEBENSMITTELZUSAMMENSETZUNG MIT DER IN DIESEM VERFAHREN HERGESTELLTEN EIGELB-PHOSPHOLIPID-ZUSAMMENSETZUNG
PROCÉDÉ DE FABRICATION D'UNE COMPOSITION DE PHOSPHOLIPIDE DE JAUNE D' OEUF PURIFIÉ, ET COMPOSITION PHARMACEUTIQUE, COMPOSITION COSMÉTIQUE OU COMPOSITION ALIMENTAIRE COMPRENANT UNE COMPOSITION DE PHOSPHOLIPIDE DE JAUNE D' OEUF PURIFIÉ OBTENUE PAR LE PROCÉDÉ

(30) Priority: 20.11.2009 JP 2009264907
(43) Date of publication of application: 26.09.2012
(73) Proprietor: Kewpie Kabushiki Kaisha (also Trading As Kewpie Corporation), Tokyo 150-0002 (JP)
(72) Inventor: NISHIMURA Ae, Fuchu-shi Tokyo 183-0034 (JP); YOSHIDA Hideto, Fuchu-shi Tokyo 183-0034 (JP)
(74) Representative: Winter, Brandl, Fürniss, Hübner, Röss, Kaiser, Polte - Partnerschaft mbB
(86) International application number: PCT/JP2010/066511
(87) International publication number: WO 2011/061995

(56) References cited:
- WO-A1-00/00038
- WO-A1-2004/014144
- JP-A- 9 000 167
- JP-A- 9 224 584
- JP-A- 2005 272 380
- "Zardi-e- Baiz Neem Brist", TKDL,, 1 January 1896 (1896-01-01), XP003029296,
- "Nuskha Bara--e Jhaeen Wa Kalaf", TKDL,, 1 January 1911 (1911-01-01), XP003029297,
- "Tila Baiza Biryan", TKDL,, 1 January 1896 (1896-01-01), XP003029298,
- "Matbookh Zardi-e- Baiz", TKDL,, 1 January 1896 (1896-01-01), XP003029299,

## Description

### Technical Field

The present invention relates to a method for producing a purified egg-yolk phospholipid composition having a reduced cholesterol content, and to the use of the purified egg-yolk phospholipid composition obtained by this method in the preparation of a pharmaceutical composition, a cosmetic composition, or a food composition.

### Background Art

An egg-yolk phospholipid has a hydrophilic group and a hydrophobic group in its molecule and has conventionally been used as an emulsifier widely in food, cosmetic, and pharmaceutical fields. In particular, an egg-yolk phospholipid having a phospholipid content of 80% or more and a neutral phospholipid content of 60% or more is excellent in emulsifiability and safety, and hence has been used as an additive for pharmaceuticals such as an emulsifier for fat emulsions or as a cosmetic raw material such as an emulsifier for skin cosmetics such as milky lotions.

When a phospholipid is extracted from egg yolk, impurities such as cholesterol and a neutral lipid derived from egg yolk are also extracted together. As a method of reducing contents of the impurities to purify an egg-yolk phospholipid composition, separation by treatment with acetone is generally known (Non Patent Literature1). When an egg-yolk phospholipid composition extracted from egg yolk is mixed with acetone, an egg-yolk phospholipid which is poorly soluble in acetone precipitates. The egg-yolk phospholipid can be purified by removing acetone having dissolved therein impurities such as a neutral lipid.

However, this method has the following problem. That is, efficiency of removal of cholesterol is low although a content of a neutral lipid can be reduced sufficiently, and hence in order to obtain a purified egg-yolk phospholipid composition having a cholesterol content reduced to a level equal to or lower than a specific level, it is necessary to repeat the treatment with acetone many times, resulting in not only costing time and money but also reducing a yield.

As a method of reducing a cholesterol content in a phospholipid, there is known a method involving: dissolving a purified phospholipid in a non-polar solvent; bringing the resultant solution into contact with silica gel; removing the silica gel; and distilling the non-polar solvent off from the solution (Patent Literature 1).

However, this method has the following problem. That is, it is difficult to obtain an effect of reducing a cholesterol content without using a purified phospholipid having a neutral lipid content of 5% or less, and it is necessary to carry out a purification treatment with acetone or the like for an egg-yolk phospholipid composition extracted from egg yolk followed by treatment with silica gel, resulting in costing time and money.

### Citation List

### Patent Literature

[PTL 1] JP 62-39593 A
[PTL 2] JP 2005-272380 A

### Non Patent Literature

[NPL 1] Ryo Nakamura (Ed.), "Science of Egg (Tamago-no-kagaku)," Asakura Publishing Co., Ltd.

### Summary of Invention

### Technical Problem

Thus, an object of the present invention is to provide a method for producing a purified egg-yolk phospholipid composition, including producing a purified egg-yolk phospholipid composition whose cholesterol content can be reduced effectively, and a pharmaceutical composition, a cosmetic composition, or a food composition including a purified egg-yolk phospholipid composition obtained by the method.

### Solution to Problem

The inventors of the present invention have made intensive studies on various conditions such as raw materials used and steps in order to achieve the above-mentioned object. As a result, the inventors have found that a purified egg-yolk phospholipid composition having a reduced cholesterol content can be obtained by carrying out treatment with a water- and alcohol-containing acetone having an acetone content of 80 vol% or more and an alcohol content of 0.3 to 3 vol% in purification of an egg-yolk phospholipid composition in a method for producing a purified egg-yolk phospholipid composition, thus completing the present invention.

That is, the present invention includes the following:
(1) a method for producing a purified egg-yolk phospholipid composition which has a phospholipid content of 80% or more and a neutral phospholipid content of 60% or more, the method including carrying out treatment with a water- and alcohol-containing acetone having an acetone content of 80 vol% or more and an alcohol content of 0.3 to 3 vol% in purification of an egg-yolk phospholipid composition which is an extract from egg-yolk with water-containing alcohol;
(2) the method for producing a purified egg-yolk phospholipid composition according to the above-mentioned item (1), in which the water content of the water- and alcohol-containing acetone is 0.3 to 6 vol%;
(3) the method for producing a purified egg-yolk phospholipid composition according to the above-mentioned items (1) to (2), in which the treatment with the water- and alcohol-containing acetone is carried out twice or more;
(4) the method for producing a purified egg-yolk phospholipid composition according to any one of the above-mentioned items (1) to (3), in which the treatment with the water- and alcohol-containing acetone is carried out under a temperature condition of -10 to 35°C;
(5) the method for producing a purified egg-yolk phospholipid composition according to any one of the above-mentioned items (1) to (4), wherein the purified egg-yolk phospholipid composition has a cholesterol content of 1.5% or less;
(6) the method for producing a purified egg-yolk phospholipid composition according to any one of the above-mentioned items (1) to (5), in which the alcohol in the water- and alcohol-containing acetone is an alcohol having 1 to 3 carbon atoms;
(7) the method for producing a purified egg-yolk phospholipid composition according to any one of the above-mentioned items (1) to (6), in which the amount of the water- and alcohol-containing acetone used in the treatment is 1 to 10 weight parts with respect to 1 weight part of the egg-yolk phospholipid composition;
(8) the use of a purified egg-yolk phospholipid composition obtained by the method according to any one of the above-mentioned items (1) to (7) in the preparation of a pharmaceutical composition;
(9) the use of a purified egg-yolk phospholipid composition obtained by the method according to any one of the above-mentioned items (1) to (7) in the preparation of a cosmetic composition; and
(10) the use of a purified egg-yolk phospholipid composition obtained by the method according to any one of the above-mentioned items (1) to (7) in the preparation of a food composition.

It should be noted that the applicants of the present invention have disclosed a method for producing a purified egg-yolk phospholipid composition with an improved color tone, the method including treating a crude egg-yolk phospholipid composition obtained from egg yolk by extraction with water-containing acetone and removing the water-containing acetone from the resultant acetone-insoluble matter (Patent Literature 2). Although a purified egg-yolk phospholipid composition obtained by the method has an improved color tone, the method has an insufficient effect of reducing a cholesterol content.

### Advantageous Effects of Invention

According to the present invention, it is possible to provide the purified egg-yolk phospholipid composition having a reduced cholesterol content, and the pharmaceutical composition, the cosmetic composition, or the food composition including the purified egg-yolk phospholipid composition, and thus a demand for a phospholipid composition is expected to further increase.

### Description of Embodiments

Hereinafter, the present invention is described in detail. It should be noted that "%" means "mass%" and "part (s) " means "part (s) by mass" in the present invention.

### 1. Purified egg-yolk phospholipid composition

The present invention is characterized by carrying out treatment with a water- and alcohol-containing acetone having an acetone content of 80 vol% or more and an alcohol content of 0.3 to 3 vol% in purification of an egg-yolk phospholipid composition which is an extract from egg-yolk with water-containing alcohol in a method for producing a purified egg-yolk phospholipid composition in order to obtain a purified egg-yolk phospholipid composition having a reduced cholesterol content.

In the present invention, the "purified egg-yolk phospholipid composition" refers to a composition having a phospholipid content of 80% or more and a neutral phospholipid content of 60% or more and also encompasses one containing a neutral lipid, cholesterol, and the like as other components. The "phospholipid" of the present invention refers to a phospholipid derived from egg yolk or a phospholipid obtained by exchanging or removing a base bonded to the phospholipid derived from egg yolk, and examples thereof include phosphatidylcholine, phosphatidylethanolamine, phosphatidylinositol, phosphatidylserine, phosphatidic acid, lysophospholipids thereof, and sphingomyelin. In addition, the "neutral phospholipid" refers to a phospholipid showing have neutral properties among the phospholipids, and examples thereof include phosphatidylcholine, lysophosphatidylcholine, and sphingomyelin.

When the purified egg-yolk phospholipid composition of the present invention is used in a pharmaceutical composition, a cosmetic composition, or a food composition, the content of cholesterol as an impurity is preferably as low as possible. Specifically, the cholesterol content of the purified egg-yolk phospholipid composition is preferably 1.5% or less, more preferably 1% or less.

### 2. Method for measuring phospholipid content, neutral phospholipid content, and cholesterol content

In the present invention, the phospholipid content, the neutral phospholipid content, and the cholesterol content are determined by measuring the purified egg-yolk phospholipid composition by a TLC-FID method. That is, an IATROSCAN (TH-10: manufactured by Mitsubishi Kagaku Iatron, Inc.) is used, and a solution obtained by dissolving 0.1 g of a sample of the resultant purified egg-yolk phospholipid composition in a chloroform:methanol (2 parts by volume:1 part by volume) solution is injected into a chromarod and developed with a chloroform:methanol:water (70 parts by volume:30 parts by volume:3 parts by volume) solution, followed by drying. Subsequently, the sample is developed with a hexane:diethyl ether: formic acid (90 parts by volume: 10 parts by volume:0.1 part by volume) solution. The lipid composition of the sample is analyzed at a scan speed of 30 to calculate the phospholipid content, the neutral phospholipid content, and the cholesterol content.

### 3. Raw material

A raw material used in the method for producing a purified egg-yolk phospholipid composition according to the present invention is not particularly limited as long as the raw material is a composition containing an egg-yolk phospholipid. In general, an egg-yolk phospholipid composition obtained from egg yolk by extraction with an organic solvent may be used, and the above-mentioned "purified egg-yolk phospholipid composition" may be used as a rawmaterial to produce a "purified egg-yolk phospholipid composition" having a further reduced cholesterol content. In addition, a product obtained by treating the egg-yolk phospholipid composition with an enzyme such as a phospholipase to hydrolyze a fatty-acid residue may be used.

Examples of the egg yolk used in the present invention include unprocessed raw egg yolk separated from bird egg such as chicken egg, frozen egg yolk produced in consideration of preservation, dried egg yolk, enzyme-treated egg yolk obtained by treating egg yolk with a protease, a phospholipase, or the like, and egg yolk obtained by treating egg yolk by a supercritical gas extraction method to reduce the cholesterol content. Inparticular, in carrying out the present invention, dried egg yolk is preferably used because as the water content of egg yolk used as a raw material becomes smaller, the amount of an extraction solvent becomes smaller, and the efficiency of removal of the extraction solvent becomes higher. The "dried egg yolk" refers to egg yolk which has a water content of 10% or less and is a food industrial product obtained by a spray-drying method, a freeze-drying method, a drum-drying method, for example, a vacuum continuous drying method, or the like. As a pretreatment, treatment with a protease, a phospholipase, or the like, or supercritical gas extraction for reducing a cholesterol content may be carried out.

### 4. Method for producing a purified egg-yolk phospholipid composition

### 4.1. Step of extracting egg-yolk phospholipid composition

In the present invention, first, an egg-yolk phospholipid composition is extracted from egg yolk used as a raw material. Specifically, in the case where dried egg-yolk is used as a raw material, an organic solvent which is a water-containing alcohol is added to the dried egg-yolk (water content: 4%), and the mixture is stirred using a stirring device capable of being rotated at high speed, such as a high-speed mixer. The mixture is filtered to separate the solid from the liquid, and the solvent is removed from the filtrate using a reduced-pressure distillation device. Thus, an egg-yolk phospholipid composition can be obtained.

Examples of the alcohol in the water-containing alcohol used in the present invention for extraction of the egg-yolk phospholipid composition include an alcohol such as methanol, ethanol, n-propanol, 2-propanol, 2-methyl-1-propanol or a denatured alcohol. Of those, a lower alcohol having 1 to 3 carbon atoms is preferred, and in view of using the composition in food applications, ethanol is more preferred. Further, in the present invention, the water-containing alcohol may be obtained by mixing water which may be industrially used, such as distilled water, in an alcohol. In general, a water-containing alcohol having a water content suitable for its purpose is used because the phospholipid content of the resultant egg-yolk phospholipid composition can be controlled by changing the water content of the alcohol.

The water content of the water-containing alcohol used for extraction of the egg-yolk phospholipid composition is preferably 0.5 to 20 vol%, more preferably 1 to 10 vol% from the standpoint of improving the extraction efficiency of the egg-yolk phospholipid composition.

In addition, the amount of the water-containing alcohol used for extraction of the egg-yolk phospholipid composition is preferably 2 to 20 parts, more preferably 5 to 10 parts relative to 1 part of dried egg yolk from the standpoint of improving the efficiency of extraction.

### 4.2. Step of treating egg-yolk phospholipid composition with water- and alcohol-containing acetone

Next, in order to increase the phospholipid content of the extracted egg-yolk phospholipid composition, the egg-yolk phospholipid composition is purified. In this process, the cholesterol content of the purified egg-yolk phospholipid composition can be reduced by carrying out treatment with a water- and alcohol-containing acetone. Specifically, the water- and alcohol-containing acetone is added to the egg-yolk phospholipid composition obtained by the above-mentioned extraction step, and the composition is dispersed homogeneously using a high-speed stirring machine or the like. The mixture is allowed to stand still to precipitate a phospholipid composition, and the supernatant solvent is removed.

The water- and alcohol-containing acetone of the present invention refers to acetone mixed with water usable for industrial applications such as distilled water and with an alcohol. In this case, examples of the alcohol include methanol, ethanol, n-propanol, 2-propanol, 2-methyl-1-propanol, and a denatured alcohol. Of those, a lower alcohol having 1 to 3 carbon atoms is preferred, and in view of using the alcohol in food applications, ethanol is more preferred.

The acetone content in the water- and alcohol-containing acetone is 80 vol% or more, preferably 90 to 99.4 vol%. This is because, when the acetone content is lower than the above-mentioned range, the yield of the purified egg-yolk phospholipid composition tends to decrease possibly owing to the fact that part of an egg-yolk phospholipid is dissolved in the water- and alcohol-containing acetone. In addition, the water- and alcohol-containing acetone of the present invention may contain an organic solvent such as hexane or heptane as long as the effect of the present invention is not impaired in addition to acetone, water, and an alcohol.

The alcohol content of the water- and alcohol-containing acetone is 0.3 to 3 vol%, preferably 0.5 to 2.5 vol%. This is because, when the alcohol content is lower than the above-mentioned range, it is difficult to reduce the cholesterol content of the purified egg-yolk phospholipid composition. Further, this is because, when the alcohol content is higher than the above-mentioned range, the yield of the purified egg-yolk phospholipid composition tends to decrease possibly owing to the fact that part of an egg-yolk phospholipid is dissolved in the water- and alcohol-containing acetone.

In addition, the water content of the water- and alcohol-containing acetone is preferably 0.3 to 6 vol%, more preferably 0.5 to 4 vol%. A water content which is lower than the above-mentioned range is not preferred because it becomes difficult to reduce the cholesterol content of the purified egg-yolk phospholipid composition. On the other hand, a water content which is higher than the above-mentioned range is not preferred because the yield of the purified egg-yolk phospholipid composition tends to decrease possibly owing to the fact that part of an egg-yolk phospholipid is dissolved in the water- and alcohol-containing acetone. In addition, the egg-yolk phospholipid composition is preferably treated with a water- and alcohol-containing acetone having a water content within the above-mentioned range because the content of a free fatty acid can be reduced effectively.

The amount of the water- and alcohol-containing acetone used in the treatment with the water- and alcohol-containing acetone is preferably 1 to 10 parts, more preferably 2 to 5 parts with respect to 1 part of the egg-yolk phospholipid composition as a raw material from the standpoint of improving the efficiency of the treatment.

It is effective to repeatedly carry out the treatment with the water- and alcohol-containing acetone because impurities such as cholesterol and a neutral lipid can be further removed. The frequency of the treatment with the water- and alcohol-containing acetone is preferably 2 or more, more preferably 3 to 5 in view of the degree of purification of the egg-yolk phospholipid composition and the cost and time for the treatment step. In addition, treatment with another solvent such as acetone or water-containing acetone may be carried out, if necessary, in the case before or after the treatment with the water- and alcohol-containing acetone, or in the case where the treatment with the water- and alcohol-containing acetone is repeatedly carried out, between the treatments.

Moreover, the treatment with the water- and alcohol-containing acetone is carried out under a temperature condition of preferably -10 to 35°C, more preferably 0 to 20°C. A temperature which is higher than the above-mentioned range is not preferred because the yield of the purified egg-yolk phospholipid composition tends to decrease. On the other hand, a temperature which is lower than the above-mentioned range is not preferred because dispersibility of the phospholipid in the treatment is deteriorated, resulting in lowering the efficiency of the treatment.

### 4.3. Drying egg-yolk phospholipid composition

The method for producing a purified egg-yolk phospholipid composition according to the present invention may include drying the egg-yolk phospholipid composition obtained by removing the supernatant solvent after the above-mentioned treatment with the water- and alcohol-containing acetone.

In this case, in the step of drying the egg-yolk phospholipid composition, the egg-yolk phospholipid composition obtained by removing the supernatant solvent is desolvated under vacuum, to thereby obtain a purified egg-yolk phospholipid composition.

### 5. Application

The present invention also relates to the use of the purified egg-yolk phospholipid composition obtained by the above-mentioned production method in the preparation of a pharmaceutical composition, a cosmetic composition, or a food composition including the purified egg-yolk phospholipid composition. Specific examples of the composition include a pharmaceutical composition such as a fat emulsion or a lipoid medicine, a cosmetic composition such as a skin milky lotion or a skin cream, a food composition such as cocoa, white stew, or a fluid diet, and intermediate raw materials used for producing the products. The purified egg-yolk phospholipid composition obtained by the above-mentioned production method has a reduced cholesterol content, and hence is more excellent in safety.

Hereinafter, the present invention is described specifically based on examples and test examples. It should be noted that the present invention is not limited thereto.

### Examples

### [Example 1]

An egg-yolk phospholipid composition was extracted from dried egg yolk (obtained by spray-drying an egg-yolk liquid and having a water content of 3%) with a water-containing ethanol (water content: 5 vol%). The resultant egg-yolk phospholipid composition had a phospholipid content of about 60%.

A mixed solvent including 192 mL of acetone, 4 mL of distilled water, and 4 mL of ethanol (water and ethanol-containing acetone having a water content of 2 vol% and an ethanol content of 2 vol%) was added to 90 g of the egg-yolk phospholipid composition, and the mixture was stirred using a high-speed stirring machine (Physcotron NS-50: manufactured by Nichion Irika, scale 30) for 5 minutes while maintaining the temperature of the mixture at 10 °C. The mixture was allowed to stand still to precipitate acetone-insoluble matter (mainly including an egg-yolk phospholipid), and decantation of the supernatant water- and ethanol-containing acetone was carried out. The operations were repeated four times, and the resultant acetone insoluble matter was desolvated under vacuum, to thereby obtain 47 g of a purified egg-yolk phospholipid composition.

The lipid composition of the resultant purified egg-yolk phospholipid composition was analyzed by a TLC-FID method, and the composition was found to have a phospholipid content of 95%, a neutral phospholipid content of 78%, and a cholesterol content of 0.5%.

### [Test Example 1]

In order to examine the influence of the water content and alcohol content of the mixed solvent used in the step of the treatment with the water- and alcohol-containing acetone on the effect of reducing the cholesterol content of the purified egg-yolk phospholipid composition, the following test was carried out. Specifically, purified egg-yolk phospholipid compositions were produced in the same manner as in Example 1, except that the water- and ethanol-containing acetone used in Example 1 was replaced by acetone solvents having the water contents and ethanol contents shown in Table 1. The lipid compositions of the resultant purified egg-yolk phospholipid compositions were analyzed in the same manner as in Example 1. It should be noted that the acetone treatment was carried out under the following conditions : acetone or a mixed solvent obtained by mixing water and/or an alcohol in acetone was used in an amount of 200 mL, and the treatment was repeated four times. In addition, all the resultant purified egg-yolk phospholipid compositions each had a phospholipid content of 80% or more and a neutral phospholipid content of 60% or more.

**[Table 1]**

| | Water content (%) | Ethanol content (%) | Cholesterol content (%) | Yield (g) |
|---|---|---|---|---|
| Sample 1 | 7 | 2 | 0.7 | 30 |
| Sample 2 | 4 | 2 | 0.7 | 42 |
| Sample 3 | 2 | 2 | 0.5 | 47 |
| Sample 4 | 0.5 | 2 | 0.9 | 48 |
| Sample 5 | 0.3 | 2 | 1.2 | 48 |
| Sample 6 | 0 | 2 | 2.1 | 48 |
| Sample 7 | 2 | 4 | 0.6 | 32 |
| Sample 8 | 2 | 3 | 0.6 | 42 |
| Sample 9 | 2 | 2.5 | 0.6 | 47 |
| Sample 10 | 2 | 0.5 | 0.9 | 47 |
| Sample 11 | 2 | 0.3 | 1.3 | 47 |
| Sample 12 | 2 | 0 | 1.7 | 47 |

Table 1 reveals that each of the purified egg-yolk phospholipid compositions obtained by the treatment with the water- and alcohol-containing acetone having a water content of 0.3 to 6 vol% and an alcohol content of 0.3 to 3 vol% had a reduced cholesterol content and was produced in a high yield, and that each of the purified egg-yolk phospholipid compositions obtained by the treatment with the water- and alcohol-containing acetone having a water content of 0.5 to 4 vol% and an alcohol content of 0.5 to 2.5 vol% had a further reduced cholesterol content and was produced in a high yield.

### [Test Example 2]

In order to examine the influence of the frequency of the treatment with the water- and alcohol-containing acetone on the effect of reducing the cholesterol content of the purified egg-yolk phospholipid composition in the step of the treatment with the water- and alcohol-containing acetone, the following test was carried out. Specifically, purified egg-yolk phospholipid compositions were obtained in the same manner as in Example 1, except that, in the method for producing a purified egg-yolk phospholipid composition of Example 1, the frequency of the treatment with the water- and alcohol-containing acetone was changed as shown in Table 2, and the same treatment was carried out using a mixed solvent including 196 mL of acetone and 4 mL of distilled water (water-containing acetone having a water content of 2 vol%) so that the frequency of the treatment with the solvent was four in total. The lipid compositions of the resultant purified egg-yolk phospholipid compositions were analyzed in the same manner as in Example 1. It should be noted that all the resultant purified egg-yolk phospholipid compositions each had a phospholipid content of 80% or more and a neutral phospholipid content of 60% or more.

**[Table 2]**

| | Frequency of treatment with water- and alcohol-containing acetone | Frequency of treatment with water-containing acetone | Cholesterol content (%) | Yield (g) |
|---|---|---|---|---|
| Sample 13 | 4 | 0 | 0.5 | 47 |
| Sample 14 | 3 | 1 | 1.0 | 47 |
| Sample 15 | 2 | 2 | 1.2 | 47 |
| Sample 16 | 1 | 3 | 1.5 | 47 |
| Sample 17 | 0 | 4 | 1.7 | 47 |

Table 2 reveals that the cholesterol content of the purified egg-yolk phospholipid composition can be reduced by treating the egg-yolk phospholipid composition with the water- and alcohol-containing acetone, and that the cholesterol content of the purified egg-yolk phospholipid composition can further be reduced by increasing the frequency of the treatment with the water- and alcohol-containing acetone.

### [Example 2]

A purified phospholipid composition (42 g) was obtained in the same manner as in Example 1, except that the treatment with the water- and ethanol-containing acetone was carried out by stirring a mixture of the egg-yolk phospholipid composition and the water- and ethanol-containing acetone for 5 minutes while maintaining the temperature of the mixture at 35°C in Example 1.

The lipid composition of the resultant purified egg-yolk phospholipid composition was analyzed in the same manner as in Example 1, and the composition was found to have a phospholipid content of 94%, a neutral phospholipid content of 77%, and a cholesterol content of 0.5%.

### [Example 3]

A purified phospholipid composition (46 g) was obtained in the same manner as in Example 1, except that the water- and ethanol-containing acetone used in Example 1 was replaced by a mixed solvent including 192 mL of acetone, 4 mL of distilled water, and 4 mL of methanol (water- and methanol-containing acetone having a water content of 2 vol% and a methanol content of 2 vol%).

The lipid composition of the resultant purified egg-yolk phospholipid composition was analyzed in the same manner as in Example 1, and the composition was found to have a phospholipid content of 96%, a neutral phospholipid content of 78%, and a cholesterol content of 0.4%.

### [Example 4]

The purified egg-yolk phospholipid composition obtained in Example 1 was used as an emulsifier to prepare a fat emulsion (one example of the pharmaceutical composition) according to the following formulation. Specifically, the purified egg-yolk phospholipid composition (Example 1) was dispersed in purified soybean oil, and the mixture was heated to 60°C. Subsequently, concentrated glycerin was added to distilled water for injection, and the mixture was heated to 60°C. The mixtures were stirred using a high-speed stirring machine (Physcotron NS-50: manufactured by Nichion Irika, scale 30) at room temperature (20 to 25°C) for 30 minutes, and emulsified homogeneously with a TK homomixer (manufactured by PRIMIX Corporation) to prepare a fat emulsion.

### [Formulation]

| | |
|---|---|
| Purified egg-yolk phospholipid composition (Example 1) | 12 (g) |
| Purified soybean oil | 200 |
| Concentrated glycerin | 25 |
| Distilled water for injection | (1,000 in total) |

### [Example 5]

The purified egg-yolk phospholipid composition obtained in Example 1 was used as an emulsifier to prepare a skin cosmetic composition according to the following formulation. Specifically, according to the following formulation, polyethylene glycol, triethanolamine, and the purifiedegg-yolkphospholipid composition (Example 1) were added to purified water and dissolved by heating, and the mixture was maintained at 70°C (aqueous phase). Subsequently, the other components were mixed and dissolved by heating, and the mixture was maintained at 70°C (oil phase). The oil phase was added to the aqueous phase to carry out preliminary emulsification, and the mixture was emulsified homogeneously with a TK homomixer (manufactured by PRIMIX Corporation). After the emulsification, the mixture was cooled to 30°C, to thereby prepare a skin cosmetic composition.

### [Formulation]

| | |
|---|---|
| Stearic acid | 25 (g) |
| Cetyl alcohol | 15 |
| Vaseline | 50 |
| Liquid paraffin | 100 |
| Polyoxyethylene (10 mol) monooleate | 20 |
| Polyethylene glycol 1500 | 30 |
| Triethanolamine | 10 |
| Purified egg-yolk phospholipid composition (Example 1) | 20 |
| Purified water | 725 |
| Flavoring agent | 5 |
| Preservative | q.s. |

### [Example 6]

The purified egg-yolk phospholipid composition obtained in Example 1 was used as an emulsifier to prepare a protein beverage (food composition) according to the following formulation. Specifically, according to the following formulation, soybean protein isolate, high-fructose corn syrup, dextrin, the purified egg-yolk phospholipid composition (Example 1), a flavoring agent, an emulsifier, and a thickening and stabilizing agent were added to purified water stirred with a TK homomixer, and the components were dissolved completely. Purified soybean oil was gradually added thereto to carry out primary emulsification. The liquid was heated to 80°C to carry out secondary emulsification using a high-pressure homogenizer (manufactured by IZUMI FOOD MACHINERY CO., LTD.), to thereby prepare a protein beverage (food composition).

### [Composition]

| | |
|---|---|
| Soybean protein isolate | 50 (g) |
| High-fructose corn syrup | 50 |
| Dextrin (DE25) | 100 |
| Purified soybean oil | 40 |
| Purified egg-yolk phospholipid composition (Example 1) | 20 |
| Flavoring agent | 5 |
| Emulsifier (sucrose fatty acid ester) | 2 |
| Thickening and stabilizing agent | 2 |
| Purified water | (1,000 in total) |

## Claims

1. A method for producing a purified egg-yolk phospholipid composition which has a phospholipid content of 80% or more and a neutral phospholipid content of 60% or more, the method comprising carrying out treatment with a water- and alcohol-containing acetone having an acetone content of 80 vol% or more and an alcohol content of 0.3 to 3 vol% in purification of an egg-yolk phospholipid composition which is an extract from egg-yolk with water-containing alcohol.

2. The method for producing a purified egg-yolk phospholipid composition according to claim 1, wherein a water content of the water- and alcohol-containing acetone is 0.3 to 6 vol%.

3. The method for producing a purified egg-yolk phospholipid composition according to claims 1 to 2, wherein the treatment with the water- and alcohol-containing acetone is carried out twice or more.

4. The method for producing a purified egg-yolk phospholipid composition according to any one of claims 1 to 3, wherein the treatment with the water- and alcohol-containing acetone is carried out under a temperature condition of -10 to 35°C.

5. The method for producing a purified egg-yolk phospholipid composition according to any one of claims 1 to 4, wherein the purified egg-yolk phospholipid composition has a cholesterol content of 1.5% or less.

6. The method for producing a purified egg-yolk phospholipid composition according to any one of claims 1 to 5, wherein the alcohol in the water- and alcohol-containing acetone is an alcohol having 1 to 3 carbon atoms.

7. The method for producing a purified egg-yolk phospholipid composition according to any one of claims 1 to 6, wherein the amount of the water- and alcohol-containing acetone used in the treatment is 1 to 10 weight parts with respect to 1 weight part of the egg-yolk phospholipid composition.

8. Use of a purified egg-yolk phospholipid composition obtained by the method of any one of claims 1 to 7 in the preparation of a pharmaceutical composition.

9. Use of a purified egg-yolk phospholipid composition obtained by the method of any one of claims 1 to 7 in the preparation of a cosmetic composition.

10. Use of a purified egg-yolk phospholipid composition obtained by the method of any one of claims 1 to 7 in the preparation of a food composition.

## Patentansprüche

1. Verfahren zur Herstellung einer gereinigten Eigelbphospholipid-Zusammensetzung, die einen Phospholipidgehalt von 80 % oder mehr und einen Gehalt an neutralem Phospholipid von 60 % oder mehr aufweist, wobei das Verfahren bei Reinigung einer Eigelbphospholipid-Zusammensetzung, die ein Extrakt von Eigelb mit wasserhaltigem Alkohol ist, Durchführen einer Behandlung mit einem wasser- und alkoholhaltigen Aceton mit einem Acetongehalt von 80 Vol.-% oder mehr und einem Alkoholgehalt von 0,3 bis 3 Vol.-% umfasst.

2. Verfahren zur Herstellung einer gereinigten Eigelbphospholipid-Zusammensetzung nach Anspruch 1, wobei der Wassergehalt des wasser- und alkoholhaltigen Acetons 0,3 bis 6 Vol.-% ist.

3. Verfahren zur Herstellung einer gereinigten Eigelbphospholipid-Zusammensetzung nach den Ansprüchen 1 bis 2, wobei die Behandlung mit dem wasser- und alkoholhaltigen Aceton zweimal oder öfters durchgeführt wird.

4. Verfahren zur Herstellung einer gereinigten Eigelbphospholipid-Zusammensetzung nach einem der Ansprüche 1 bis 3, wobei die Behandlung mit dem wasser- und alkoholhaltigen Aceton unter Temperaturbedingungen von -10 bis 35°C durchgeführt wird.

5. Verfahren zur Herstellung einer gereinigten Eigelbphospholipid-Zusammensetzung nach einem der Ansprüche 1 bis 4, wobei die gereinigte Eigelbphospholipid-Zusammensetzung einen Cholesteringehalt von 1,5 % oder weniger hat.

6. Verfahren zur Herstellung einer gereinigten Eigelbphospholipid-Zusammensetzung nach einem der Ansprüche 1 bis 5, wobei der Alkohol in dem wasser- und alkoholhaltigen Aceton ein Alkohol mit 1 bis 3 Kohlenstoffatomen ist.

7. Verfahren zur Herstellung einer gereinigten Eigelbphospholipid-Zusammensetzung nach einem der Ansprüche 1 bis 6, wobei die Menge des bei der Behandlung verwendeten wasser- und alkoholhaltigen Acetons 1 bis 10 Gewichtsteile auf 1 Gewichtsteil der Eigelbphospholipid-Zusammensetzung beträgt.

8. Verwendung einer durch das Verfahren gemäß einem der Ansprüche 1 bis 7 erhaltenen gereinigten Eigelbphospholipid-Zusammensetzung bei der Herstellung einer pharmazeutischen Zusammensetzung.

9. Verwendung einer durch das Verfahren gemäß einem der Ansprüche 1 bis 7 erhaltenen gereinigten Eigelbphospholipid-Zusammensetzung bei der Herstellung einer kosmetischen Zusammensetzung.

10. Verwendung einer durch das Verfahren gemäß einem der Ansprüche 1 bis 7 erhaltenen gereinigten Eigelbphospholipid-Zusammensetzung bei der Herstellung einer Nahrungsmittelzusammensetzung.

## Revendications

1. Procédé de production d'une composition de phospholipides de jaune d'oeuf purifiés qui a une teneur en phospholipides de 80 % ou supérieure et une teneur en phospholipides neutres de 60 % ou supérieure, le procédé comprenant la réalisation d'un traitement avec une acétone contenant de l'eau et de l'alcool ayant une teneur en acétone de 80 % en volume ou plus et une teneur en alcool de 0,3 à 3 % en volume dans la purification d'une composition de phospholipides de jaune d'oeuf qui est un extrait issu de jaune d'oeuf avec de l'alcool contenant de l'eau.

2. Procédé de production d'une composition de phospholipides de jaune d'oeuf purifiés selon la revendication 1, dans lequel la teneur en eau de l'acétone contenant de l'eau et de l'alcool est de 0,3 à 6 % en volume.

3. Procédé de production d'une composition de phospholipides de jaune d'oeuf purifiés selon les revendications 1 et 2, dans lequel le traitement avec l'acétone contenant de l'eau et de l'alcool est réalisé deux fois ou plus.

4. Procédé de production d'une composition de phospholipides de jaune d'oeuf purifiés selon l'une quelconque des revendications 1 à 3, dans lequel le traitement avec l'acétone contenant de l'eau et de l'alcool est réalisé dans des conditions de température de -10 à 35 °C.

5. Procédé de production d'une composition de phospholipides de jaune d'oeuf purifiés selon l'une quelconque des revendications 1 à 4, dans lequel la composition de phospholipides de jaune d'oeuf purifiés a une teneur en cholestérol de 1,5 % ou inférieure.

6. Procédé de production d'une composition de phospholipides de jaune d'oeuf purifiés selon l'une quelconque des revendications 1 à 5, dans lequel l'alcool dans l'acétone contenant de l'eau et de l'alcool est un alcool comportant 1 à 3 atomes de carbone.

7. Procédé de production d'une composition de phospholipides de jaune d'oeuf purifiés selon l'une quelconque des revendications 1 à 6, dans lequel la quantité de l'acétone contenant de l'eau et de l'alcool utilisée dans le traitement est de 1 à 10 parties en poids par rapport à 1 partie en poids de la composition de phospholipides de jaune d'oeuf.

8. Utilisation d'une composition de phospholipides de jaune d'oeuf purifiés obtenue par le procédé selon l'une quelconque des revendications 1 à 7 dans la préparation d'une composition pharmaceutique.

9. Utilisation d'une composition de phospholipides de jaune d'oeuf purifiés obtenue par le procédé selon l'une quelconque des revendications 1 à 7 dans la préparation d'une composition cosmétique.

10. Utilisation d'une composition de phospholipides de jaune d'oeuf purifiés obtenue par le procédé selon l'une quelconque des revendications 1 à 7 dans la préparation d'une composition alimentaire.
